Europäisches Patentamt

European Patent Office

Office européen des brevets

(19)

(11) Veröffentlichungsnummer: **0 050 288**
**B1**

(12)

# EUROPÄISCHE PATENTSCHRIFT

(45) Veröffentlichungstag der Patentschrift:
16.01.85

(21) Anmeldenummer: 81108216.3

(22) Anmeldetag: **12.10.81**

(51) Int. Cl.⁴: **B 01 D 13/00**, F 26 B 5/00,
B 65 D 65/38, B 32 B 33/00,
F 24 F 6/00, A 01 F 25/13 //
D06N3/04, A61G15/00

(54) **Verwendung von wasserdampfdurchlässigen Folien aus Polyvinylalkohol.**

(30) Priorität: 18.10.80 DE 3039386

(43) Veröffentlichungstag der Anmeldung:
28.04.82 Patentblatt 82/17

(45) Bekanntmachung des Hinweises auf die Patenterteilung:
16.01.85 Patentblatt 85/3

(84) Benannte Vertragsstaaten:
AT BE CH DE FR GB IT LI NL SE

(56) Entgegenhaltungen:
EP - A - 0 004 587
DE - A - 2 109 940
DE - A - 2 523 287
FR - A - 1 320 683
FR - A - 1 578 813
FR - A - 2 365 437
US - A - 2 546 705
US - A - 3 035 060

FINCH, C.A. (Ed.): "polyvinyl alcohol; properties and applications" Wiley, 1973, London, G.B.

(73) Patentinhaber: **HOECHST AKTIENGESELLSCHAFT,
Postfach 80 03 20, D-6230 Frankfurt am Main 80 (DE)**

(72) Erfinder: **Zimmermann, Wolgang, im Stückes 48,
D-6233 Kelkheim (Taunus) (DE)**
Erfinder: **Harréus, Albrecht, Dr.,
Philipp-Kremer-Strasse 16, D-6233 Kelkheim (Taunus)
(DE)**
Erfinder: **Gutte, Richard, Heimchenweg 52,
D-6230 Frankfurt am Main 80 (DE)**

## Beschreibung

Die Erfindung bezieht sich auf eine Verwendung von wasserdampfdurchlässigen Folien aus Polyvinylalkohol.

Es ist bekannt, dass Polyvinylalkohol (PVAL) gute Wasserlöslichkeit aufweist und dass diese Eigenschaft von der Esterzahl abhängig ist und durch chemische Nachbehandlung, z.B. durch partielle Acetalisierung, variiert werden kann. Ebenfalls bekannt ist die thermoplastische Verformbarkeit des PVAL, der z.B. durch Pressen oder Extrudieren zu Folien verarbeitet wird, die in kaltem Wasser löslich sind; solche Folien dienen bespielsweise zur Verpackung von Farbstoffen, Giftstoffen, Schädlingsbekämpfungsmitteln und Waschmitteln (vgl. Kunststoff-Handbuch, Band XI (1971), Seiten 509 bis 516). Die Wasserlöslichkeit derartiger Folien kann durch mechanische Behandlung, z.B. Verstreckung, oder durch chemische Nachbehandlung, z.B. Acetalisierung, beeinflusst werden.

Ein Verfahren zur Veränderung des Wassergehaltes einer Flüssigkeit mit Hilfe eines aus Kunststoffmaterial, z.B. Polyvinylalkohol, hergestellten semipermeablen Films, der in dem Lösungsmittel unlöslich ist, ist aus FR-A-2 365 437 bekannt. Es ist auch bereits ein Verfahren zum Konzentrieren von aromahaltigen wässrigen Flüssigkeiten mittels einer Membran aus PVAL, wobei der PVAL flüssiges Wasser permeieren lässt, bekannt (vgl. FR-A-1 578 813).

Aufgabe der Erfindung ist nun die Be- oder Entfeuchtung von Feststoffen, Flüssigkeiten oder Gasen unter Einsatz einer Folie aus PVAL, die als Trennfolie zwischen dem Material, das be- oder entfeuchtet werden soll, und dessen Umgebung dient.

Die Erfindung betrifft die Verwendung von wasserdampfdurchlässigen Folien als Material für wasserdampfdurchlässige Trennschichten, dadurch gekennzeichnet, dass die verwendeten Folien aus Polyvinylalkohol bestehen, der eine Esterzahl von 0 bis 200 mg KOH/g aufweist und dessen 4-gewichtsprozentige wässrige Lösung bei einer Temperatur von 20 °C eine Viskosität von 4 bis 100 mPa·s zeigt, wobei die Folien bei Temperaturen unterhalb von 80 °C wasserunlöslich und für flüssiges Wasser undurchlässig sind.

Die erfindungsgemässe Verwendung dient zur Veränderung des Wassergehalts von wasseraufnehmendem oder wasserabgebendem Material unter Einsatz einer Folie, die dieses Material ganz oder teilweise von dem angrenzenden Raum trennt und unter Normalbedingungen für flüssiges Wasser undurchlässig, für gasförmiges Wasser jedoch durchlässig ist. Diese Folie eignet sich zur Trocknung oder Befeuchtung von beliebigem Material, das fest, flüssig oder gasförmig sein kann. Wesentliches Merkmal der Erfindung ist dabei die Verwendung einer PVAL-Folie für alle Einsatzgebiete, bei denen es auf die Be- und Entfeuchtung bestimmter Materialien ankommt, wobei die Verdunstung des Wassers unter Ausnutzung eines Wasserdampfdruck-Gefälles erfolgt.

Die erfindungsgemässe Verwendung ist insbesondere eine Methode zur Trocknung von wasserhaltigem Material unter sehr schonenden Bedingungen, d.h. ohne Anwendung höherer Temperaturen.

Die erfindungsgemäss verwendete Folie wird hergestellt aus einem Polyvinylalkohol, der eine Esterzahl von 0 bis 200 mg KOH/g, vorzugsweise 10 bis 150 mg KOH/g, aufweist und dessen 4-gewichtsprozentige wässrige Lösung bei einer Temperatur von 20 °C eine Viskosität von 4 bis 100 mPa·s, vorzugsweise 10 bis 60 mPa·s, zeigt. Die Folie ist bei einer Temperatur unterhalb 80 °C, insbesondere unterhalb 60 °C und vorzugsweise im Bereich von 0 bis 50 °C, in Wasser unlöslich.

Die Wasserlöslichkeit der Folie kann mechanisch, vorzugsweise durch biaxiale Verstreckung, oder chemisch, vorzugsweise durch partielle Vernetzung, gesteuert werden. Als Vernetzungsmittel dienen dabei Verbindungen, die mit den Hydroxylgruppen des PVAL reagieren. Insbesondere eignen sich bifunktionelle Verbindungen wie Bisepoxide, Diisocyanate sowie insbesondere Formaldehyd Formaldehyd abspaltende Verbindungen und Dialdehyde, z.B. Glyoxal oder Terephthalaldehyd.

Als Ausgangsmaterial zur Herstellung der Folie eignet sich insbesondere ein thermoplastisch verarbeitbares, weichmacherhaltiges, rieselfähiges, nicht klebendes PVAL-Granulat; dieses besteht zu mindestens 70 Gewichtsprozent, vorzugsweise mindestens 90 Gewichtsprozent, aus Partikeln mit einem Durchmesser von 0,8 bis 4 mm, in denen ein Weichmacher und gegebenenfalls eine feinteilige, in Wasser lösliche oder dispergierbare, hochmolekulare organische Verbindung homogen verteilt vorliegen. Dieses Granulat wird in bekannter Weise dadurch hergestellt, dass 100 Gewichtsteile eines trockenen PVAL-Granulats, das zu mindestens 70 Gewichtsprozent aus Partikeln mit einem Durchmesser von 0,4 bis 4 mm besteht, mit 5 bis 50 Gewichtsteilen eines Weichmachers — gegebenenfalls in Gegenwart von 1 bis 15 Gewichtsteilen einer feinteiligen, in Wasser löslichen oder dispergierbaren, hochmolekularen organischen Verbindung, die aus Partikeln mit Durchmessern von höchstens 300 μm besteht — in Gegenwart einer solchen Menge Wasser, die unter Normalbedingungen zur Lösung des PVAL nicht ausreicht, intensiv und homogen miteinander vermischt werden; während des Mischvorgangs wird die Temperatur des Gemischs derart erhöht und wieder gesenkt, dass die PVAL-Partikel quellen und vorübergehend agglomerieren (vgl. europäische Anmeldungsveröffentlichung Nr. 4587).

Die erfindungsgemäss verwendete Folie wird durch übliche Verfahren hergestellt, insbesondere nach dem Giessverfahren, Breitschlitz-Extrusionsverfahren oder vorzugsweise Blasextrusionsverfahren. Die Dicke der Folie liegt üblicherweise im Bereich von 10 bis 200 μm, vorzugsweise 30 bis 100 μm.

Unter der Voraussetzung, dass ein Wasserdampfdruck-Gefälle vorhanden ist, lässt sich jedes beliebige Material, das ganz oder teilweise mit der erfindungsgemässen PVAL-Folie umgeben oder ganz oder teilweise durch die Folie von dem an das Material angrenzenden Raum getrennt werden kann, befeuchten oder entfeuchten.

Für Trocknungszwecke empfiehlt sich, das Material in einem verschlossenen Behälter, z.B. einem Beutel oder Schlauch, aus der PVAL-Folie aufzubewahren. Oft genügt es aber auch, das Material lediglich oberflächlich mit der Folie abzudecken, wobei der seitliche Luftzutritt den Trocknungsvorgang nicht beeinträchtigt. Eine solche Trocknung kann im Freien durchgeführt werden, und eine äusserliche Benetzung der Folie durch Tau oder Regen ist auf die Trocknung des mit der Folie abgedeckten Materials ohne Einfluss.

Pflanzliches Material ist für die Trocknung nach dem erfindungsgemässen Verfahren besonders geeignet, z.B. Drogen (Blätter, Blüten, Früchte, Wurzeln), Genussmittel (Kaffee, Tee, Tabak) sowie Gewürze, Hölzer und Gras. In gleicher Weise lassen sich auch Nahrungsmittel auf pflanzlicher oder tierischer Grundlage sowie Arzneimittel mit pflanzlichen oder chemischen Wirkstoffen schonend trocknen. Etwaige in dem Material enthaltene Aromastoffe werden dabei von der Folie weitgehend zurückgehalten. Auch Baustoffe wie Mörtel, Gips und Zement können durch Abdeckung mit der PVAL-Folie getrocknet und gegen Regen und Spritzwasser geschützt werden.

Ferner bietet die erfindungsgemässe Folie die Möglichkeit, wässrige Lösungen oder Dispersionen von anorganischen oder organischen Substanzen durch Wasserentzug zu konzentrieren. Hierbei ist es zweckmässig, die PVAL-Folie in Form eines Schlauches anzuwenden, durch den die Lösung oder Dispersion geleitet wird. Diese Methode ist in gleicher Weise für Wasser-in-Öl-Emulsionen und Öl-in-Wasser-Emulsionen anwendbar. Wasserhaltige Lösemittel, z.B. Methanol, Ethanol oder Aceton, werden vorzugsweise dadurch entwässert, dass sie eine Zeitlang in einem zugeschweissten Beutel aus der PVAL-Folie an der Luft mit geringer relativer Feuchtigkeit aufbewahrt werden.

Eine weitere Methode der Trocknung mit Hilfe der PVAL-Folie besteht darin, dass ein Trocknungsmittel, z.B. Kieselsäuregel, das in einem Beutel aus der PVAL-Folie eingeschweisst ist, mit dem zu trocknenden Gut, das sich in einem geschlossenen Behälter befindet, in Kontakt gebracht wird. Auf diese Weise lassen sich sowohl feste Substanzen und Gegenstände als auch Flüssigkeiten und Gase trocknen. Vor allem hygroskopische Substanzen und feuchtigkeitsempfindliche Güter können mit Hilfe der erfindungsgemässen Folie getrocknet und trocken gehalten werden. Das gleiche gilt für technisch genutzte Gegenstände und Apparate, z.B. Waagen, elektrische oder optische Geräte und elektronische Bauteile, die wasserempfindlich sind.

Die erfindungsgemässe Folie ist vorzugsweise an Gegenständen im Sanitär- oder Hygienebereich anwendbar. Beispielsweise kann die PVAL-Folie mit einer porösen Schicht aus Fasermaterial, z.B. aus Faservlies und/oder Watte, verbunden werden, wodurch flüssigwasserfeste, aber wasserdampfdurchlässige Laminate erhalten werden, die für die Herstellung von Wundverbänden, Windeln und Krankenbett-Unterlagen geeignet sind.

Ferner ist die erfindungsgemäss verwendete Folie in Form eines mehrschichtigen Laminats einsetzbar, das die Folie als Mittelschicht enthält, und zwar insbesondere auf dem Kunstledersektor zur Herstellung von wasserdampfdurchlässigen, aber flüssigwasserfesten Laminaten, vorzugsweise als Zwischenschicht zwischen perforierten Weich-PVC-Platten.

Die Veränderung des Wassergehalts der Luft von Räumen, die von Menschen benutzt werden, insbesondere Wohnräumen und Arbeitsräumen, ist eine besondere Variante der erfindungsgemässen Verwendung. Dabei besteht die Veränderung des Wassergehalts in Abhängigkeit von den Anforderungen des Einzelfalls in einer Befeuchtung oder Entfeuchtung des Raumes. Für die gezielte Befeuchtung eines Raumes ist die Anwendung der PVAL-Folie in Form eines Schlauchsystems von Vorteil; dabei verdunstet das im Schlauch enthaltene Wasser langsam durch die Schlauchwand. Diese Methode der Raumbefeuchtung hat den Vorteil, dass die im Leitungswasser enthaltenen Härtebildner sich nicht an der Innenwand des Schlauches absetzen, sondern sich am Boden sammeln und durch Spülen des Schlauches mit Frischwasser einfach entfernt werden können.

Die folgenden Beispiele zeigen einige Anwendungen der erfindungsgemässen Folie. Prozentangaben beziehen sich jeweils auf das Gewicht.

Beispiel 1

Aus einem handelsüblichen Polyvinylalkohol-Granulat, das 20% Glycerin als Weichmacher enthält, wird durch Blasextrusion eine Folie mit einer Dicke von 30 µm hergestellt. Der PVAL hat eine Esterzahl von 20 mg KOH/g, ist bis zu einer Temperatur von 50 °C in Wasser unlöslich, und die 4prozentige wässrige Lösung des PVAL hat eine Viskosität von 20 mPa·s.

3 gleiche Schalen mit einem Durchmesser von 7 cm und einer Höhe von 3 cm werden jeweils mit der gleichen Menge Wasser gefüllt. 2 dieser Schalen werden hermetisch mit der oben beschriebenen PVAL-Folie verschlossen; die dritte Schale wird für einen Blindversuch verwendet. Die 3 Schalen werden dann in einen Klimaraum (23 °C, 50% relative Luftfeuchtigkeit) gebracht, wobei eine der mit der Folie verschlossenen Schalen umgedreht auf ein Gitter gestellt wird, so dass die Folie auf der gesamten Fläche mit dem Wasser in Kontakt ist. Im Blindversuch ist das Wasser nach 7 Tagen verdunstet. Das Wasser in der umgekehrt stehenden Schale ist nach 8 Tagen und das Wasser in der normal mit der Folie abgedeckten Schale ist nach 10 Tagen verdunstet.

Beispiel 2

a) Frische Tabakblätter werden einzeln in eine Folie der in Beispiel 1 beschriebenen Art eingeschweisst und in einem Klimaraum (23 °C, 50% relative Luftfeuchtigkeit) aufgehängt. Nach 10 Tagen beträgt das Gewicht der Tabakblätter im Mittel noch 66% des Ausgangswertes.

b) Frische Tabakblätter werden einzeln in Beuteln, hergestellt aus der in Beispiel 1 beschriebenen Folie, eingelegt, und die Beutel werden mit der Öffnung nach unten im Freien aufgehängt. Nach 10 Tagen beträgt das Gewicht der Tabakblätter im Mittel 50% des Ausgangswertes.

Beispiel 3

a) Ein Haufen frisch gemähtes Gras mit einem Wassergehalt von 80% wird mit einer Folie der in Beispiel 1 beschriebenen Art abgedeckt 4 Wochen liegen gelassen. Danach beträgt der Wassergehalt des Grases nur noch 30%.

b) Frisch gemähtes Gras mit einem Wassergehalt von 80% wird in einem Sack aus einer Folie der in Beispiel 1 beschriebenen Art eingeschweisst. Nach einer Lagerung von 4 Wochen zeigt das Gras einen Wassergehalt von 25%.

Beispiel 4

In ein Reagenzglas werden 50 g Ätznatron-Tabletten gefüllt, und auf diese Tabletten wird ein Beutel aus einer Folie der in Beispiel 1 beschriebenen Art gelegt, der mit getrocknetem Kieselsäuregel gefüllt ist. Das Reagenzglas wird dann mit einem Wattebausch verschlossen und in einen Klimaraum (23 °C, 50% relative Luftfeuchtigkeit) gebracht. Nach 5 Wochen sind die Tabletten noch völlig trocken und rieselfähig.

Beispiel 5

In einen Beutel aus einer Folie der in Beispiel 1 beschriebenen Art wird ein Gemisch aus 90% Aceton und 10% Wasser eingefüllt; der Beutel wird luftdicht verschlossen und in einen Klimaraum (23 °C, 50% relative Luftfeuchtigkeit) gehängt. Nach einer Lagerzeit von 3 Wochen beträgt der Wassergehalt des Gemischs nur noch 3%.

Beispiel 6

In einen Beutel aus einer Folie der in Beispiel 1 beschriebenen Art wird eine 10-prozentige Rohrzuckerlösung eingefüllt; der Beutel wird zugeschweisst und in einen Klimaraum (23 °C, 50% relative Luftfeuchtigkeit) gehängt. Nach einer Lagerzeit von 1 Woche wird die Lösung sirupös, und nach 3 weiteren Tagen beginnt der Zucker im Beutel zu kristallisieren.

Beispiel 7

Auf eine Folie der in Beispiel 1 beschriebenen Art wird zunächst eine Zellstoffwatteschicht und darauf eine Schicht aus Polyesterfaservlies aufgebracht. Das resultierende Laminat dient als Krankenbettunterlage, die sehr saugfähig ist und aufgesogene Feuchtigkeit in Form von Wasserdampf allmählich nach unten abgibt.

Beispiel 8

In einem Wohnraum wird an einem Holzrahmen ein Schlauch befestigt, der eine Gesamtlänge von 5 m und einen Durchmesser von 2 cm aufweist und ein geschlossenes System bildet. Der Schlauch besteht aus einer Folie der in Beispiel 1 beschriebenen Art. Mit Hilfe einer Pumpe wird Wasser kontinuierlich langsam durch den Schlauch geführt. Hinter dem Schlauchsystem befindet sich ein Ventilator, der Luft durch das System in den Raum bläst. Das verdunstete Wasser wird von Zeit zu Zeit ersetzt. Die Raumbefeuchtung wird mehrere Monate lang ohne Unterbrechung durchgeführt, ohne dass sich Verkrustungen an den Schlauchwänden bilden.

Beispiel 9

Eine Folie der in Beispiel 1 beschriebenen Art wird durch Pressen bei einer Temperatur von 150 °C beidseitig mit einer 1 mm dicken Platte aus perforiertem Weich-PVC verbunden. Der resultierende Verbund ist für flüssiges Wasser undurchlässig, für Wasserdampf aber durchlässig, und wird zur Herstellung von Artikeln aus Kunstleder verwendet.

Beispiel 10

Zwei Faserlederplatten im Format DIN A 6 und mit einer Dicke von 0,5 mm werden einseitig mit einer handelsüblichen Vinylacetat/Ethylen-Copolymer-Dispersion in einer Menge von 100 g/m² beschichtet. Zwischen die beschichteten Seiten der Faserlederplatten wird eine Folie der im Beispiel 1 beschriebenen Art eingelegt. Der so erhaltene lose Verbund wird in einer hydraulischen Presse bei einem Pressdruck von 20 bar 10 min lang verpresst.

Die Wasserdampfdurchlässigkeit des Faserlederverbundes wird wie in Beispiel 1 beschrieben untersucht. Dazu wird der Verbund auf den Rand der Schale, die 10 g Wasser enthält, geklebt. Die gesamte Wassermenge ist nach 11 Tagen aus der mit dem Verbund abgedeckten Schale verdunstet.

**Patentansprüche**

1. Verwendung von wasserdampfdurchlässigen Folien als Material für wasserdampfdurchlässige Trennschichten, dadurch gekennzeichnet, dass die verwendeten Folien aus Polyvinylalkohol bestehen, der eine Esterzahl von 0 bis 200 mg KOH/g aufweist und dessen 4-gewichtsprozentige wässrige Lösung bei 20 °C eine Viskosität von 4 bis 100 mP·s zeigt, wobei die Folien bei Temperaturen unterhalb von 80 °C wasserunlöslich und für flüssiges Wasser undurchlässig sind.

2. Verwendung von wasserdampfdurchlässigen Folien als Material für wasserdampfdurchlässige Trennschichten, dadurch gekennzeichnet, dass die verwendeten Folien aus Polyvinylalkohol bestehen, der eine Esterzahl von 0 bis 200 mg KOH/g aufweist und dessen 4-gewichtsprozentige wässrige Lösung bei 20 °C eine Viskosität von 4 bis 100 mPa·s zeigt, wobei die Folien bei Temperaturen im Bereich von 0 bis 50 °C wasserunlöslich und für flüssiges Wasser undurchlässig sind.

3. Verwendung von Folien nach Ansprüchen 1 oder 2, dadurch gekennzeichnet, dass sie aus weichmacherhaltigem Polyvinylalkohol bestehen.

4. Verwendung von Folien nach Ansprüchen 1 bis 3, dadurch gekennzeichnet, dass die Foliendicke 10 bis 200 µm beträgt.

5. Verwendung von Folien nach Ansprüchen 1 bis 4 zur Veränderung des Wassergehaltes von pflanzlichem Material.

6. Verwendung von Folien nach Ansprüchen 1 bis 4 zur Veränderung des Wassergehaltes von Nahrungsmitteln, Genussmitteln, Arzneimitteln oder Futtermitteln.

7. Verwendung von Folien nach Ansprüchen 1 bis 4 zur Veränderung des Wassergehaltes von Baustoffen.

8. Verwendung von Folien nach Ansprüchen 1 bis 4 zur Veränderung des Wassergehaltes von hygroskopischen Substanzen.

9. Verwendung von Folien nach Ansprüchen 1 bis 4 zur Veränderung des Wassergehaltes von wässrigen Lösungen oder wässrigen Dispersionen.

10. Verwendung von Folien nach Ansprüchen 1 bis 4 als Schutz für wasserempfindliche, technisch nutzbare Gegenstände und Apparate.

11. Verwendung von Folien nach Ansprüchen 1 bis 4 zur Veränderung des Feuchtigkeitsgehaltes der Raumluft von Wohn-, Arbeits- oder Nutzräumen.

12. Verwendung von Folien nach Ansprüchen 1 bis 4 als Trennschicht im Verbund mit porösen Schichten aus Fasermaterialien.

13. Verwendung von Folien nach Ansprüchen 1 bis 4 als Trennschicht in mehrschichtigen Laminaten aus perforierten Materialien.

## Revendications

1. Utilisation de feuilles perméables à la vapeur d'eau en tant que matériau pour couches de séparation perméables à la vapeur d'eau, caractérisée en ce que les feuilles utilisées sont constituées de poly(alcool vinylique) qui présente un indice d'estérification de 0 à 200 mg KOH/g et dont la solution aqueuse à 4% en poids présente à 20 °C une viscosité de 4 à 100 mPa·s, les feuilles, à des températures inférieures à 80 °C, étant imperméables à l'eau liquide et insolubles dans l'eau.

2. Utilisation de feuilles perméables à la vapeur d'eau en tant que matériau pour couches de séparation perméables à la vapeur d'eau, caractérisée en ce que les feuilles utilisées sont constituées de poly(alcool vinylique) qui présente un indice d'estérification de 0 à 200 mg KOH/g et dont la solution aqueuse à 4% en poids présente à 20 °C une viscosité de 4 à 100 mPa·s, les feuilles, à des températures comprises entre 0 et 50 °C, étant imperméables à l'eau liquide et insolubles dans l'eau.

3. Utilisation de feuilles selon l'une des revendications 1 ou 2, caractérisée en ce qu'elles sont constituées de poly(alcool vinylique) contenant un plastifiant.

4. Utilisation de feuilles selon l'une des revendications 1 à 3, caractérisée en ce que l'épaisseur des feuilles est de 10 à 200 µm.

5. Utilisation de feuilles selon l'une des revendications 1 à 4 pour modifier la teneur en eau de matières végétales.

6. Utilisation de feuilles selon l'une des revendications 1 à 4 pour modifier la teneur en eau de produits alimentaires, de stimulants, de médicaments ou de produits pour l'alimentation animale.

7. Utilisation de feuilles selon l'une des revendications 1 à 4 pour modifier la teneur en eau de matériaux de construction.

8. Utilisation de feuilles selon l'une des revendications 1 à 4 pour modifier la teneur en eau de substances hygroscopiques.

9. Utilisation de feuilles selon l'une des revendications 1 à 4 pour modifier la teneur en eau de solutions aqueuses ou de dispersions aqueuses.

10. Utilisation de feuilles selon l'une des revendications 1 à 4 en tant que protection pour objets et appareillages à utilisation technique et sensibles à l'eau.

11. Utilisation de feuilles selon l'une des revendications 1 à 4 pour modifier la teneur en humidité de l'air ambiant des locaux d'habitation, industriels ou utilitaires.

12. Utilisation de feuilles selon l'une des revendications 1 à 4 en tant que couche de séparation, en combinaison avec des couches poreuses en un matériau en fibres.

13. Utilisation de feuilles selon l'une des revendications 1 à 4 en tant que couche de séparation dans des stratifiés multicouches en matériaux perforés.

## Claims

1. Use of films permeable to water vapor as a separator material permeable to water vapor, which comprises using films made of polyvinyl alcohol, which has an ester number of between 0 and 200 mg of KOH/g and the 4 percent strength by weight aqueous solution of which exhibits a viscosity of 4 to 100 mPa·s at a temperature of 20 °C, whereby the films are water-insoluble at temperatures below 80 °C and impermeable to liquid water.

2. use of films permeable to water vapor as a separator material permeable to water vapor, which comprises using films made of polyvinyl alcohol, which has an ester number of between 0 and 200 mg of KOH/g and the 4 percent strength by weight aqueous solution of which exhibits a viscosity of 4 to 100 mPa·s at a temperature of 20 °C, whereby the films are water-insoluble at temperatures in the range of from 0 to 50 °C and impermeable to liquid water.

3. Use of films as claimed in claims 1 or 2, characterized in that the films consist of a polyvinyl alcohol containing a plasticizer.

4. Use of films as claimed in claims 1 to 3, characterized in that the thickness of the films is in the range of from 10 to 200 µm.

5. Use of films as claimed in claims 1 to 4 to vary the water content of vegetable material.

6. Use of films as claimed in claims 1 to 4 to vary the water content of foodstuffs, luxuries, remedies or feeding stuffs.

7. Use of films as claimed in claims 1 to 4 to vary the water content of building materials.

8. Use of films as claimed in claims 1 to 4 to vary the water content of hygroscopic substances.

9. Use of films as claimed in claims 1 to 4 to vary the water content of aqueous solutions or aqueous dispersions.

10. Use of films as claimed in claims 1 to 4 to protect water-sensitive technical articles and apparatuses.

11. Use of films as claimed in claims 1 to 4 to vary the moisture content of dwelling rooms, working rooms or useful rooms.

12. Use of films as claimed in claims 1 to 4 as a separator layer in connection with porous layers of fiber materials.

13. Use of films as claimed in claims 1 to 4 as a separator layer in multi-layer laminates prepared from perforated materials.